# EUROPEAN PATENT APPLICATION

(11) **EP 4 353 224 A1**
(43) Date of publication of application: **17.04.2024**
(21) Application number: 23202586.6
(22) Date of filing: 10.10.2023
(51) Int. Cl.: A61K 31/10, A61K 31/337, A61K 31/407, A61K 31/4745, A61K 31/7032, A61K 31/704, A61K 31/7048, A61K 33/243, A61K 38/14, A61K 45/06, A61P 35/00

(54) **PHARMACEUTICAL COMPOSITION COMPRISING A SULFOQUINOVOSYLACYLPROPANEDIOL DERIVATE FOR TREATING CANCER**

(30) Priority: 11.10.2022 US 202263414955 P
(71) Applicant: M.T.3, Inc., Tokyo 107-0062 (JP); Colorado State University Research Foundation, Fort Collins, CO 80526 (US)
(72) Inventor: KATO, Takamitsu, Fort Collins, 80526 (US); MAEDA, Junko, Fort Collins, 80526 (US); HAGA, Tomohiro, Tokyo, 107-0062 (JP); FUKUHARA, Takaomi, Tokyo, 107-0062 (JP)
(74) Representative: Drywood, Rosalind Aerona

(57) **Abstract**

There is provided with a pharmaceutical composition for cancer treatment to be administered to a patient with cancer in combination with an anticancer agent, the pharmaceutical composition. A compound is represented by General Formula (I) or a pharmaceutically acceptable salt thereof: 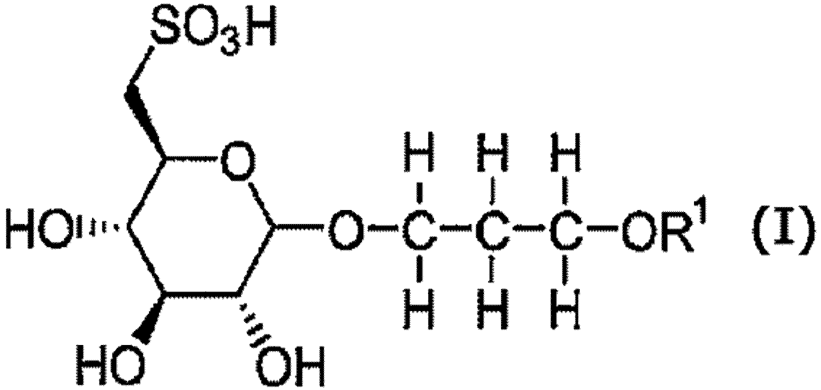 where R¹ is an acyl residue from a fatty acid.

## Description

### BACKGROUND OF THE INVENTION

### Field of the Invention

The present invention relates to a pharmaceutical composition.

### Description of the Related Art

As the life span of humans and pet animals such as dogs and cats has been extended due to advances in medical treatment, the cancer death rate has been increasing. A radiation therapy as well as an operative therapy and a chemotherapy are employed as a method for cancer treatment, and there is always a demand for advances in these treatment methods.

A technique for administering, to a patient, a radiosensitizing agent for improving the therapeutic effect of radiation treatment as a pharmaceutical composition has been studied. Known examples of the radiosensitizing agent include 2-nitroimidazol derivatives (Patent Literature 1: Japanese Patent Laid Open No. 2007-302609), which improve the radiation sensitivity of hypoxic cells, and 5-iododeoxyuridine (Patent Literature 2: Japanese Patent Laid Open No. 2002-536341), which improves radiation sensitivity through incorporation into DNA. In recent years, a sulfoquinovosylacylpropanediol derivative (Patent Literature 3: International Publication No. 2009/14101) has also been reported as a novel radiosensitizing agent. Patent Literature 3 discloses that angiogenesis was inhibited in an in-vitro test by using αSQAP C10:0, αSQAP C14:0, αSQAP C18:0, αSQAP C22:0, βSQAP C18:0, and βSQAP C18:1 in combination with radiation, and that an increase in a tumor volume was suppressed by using αSQAP C18:0 in combination with radiation in a mouse to which human esophageal squamous cell carcinoma or human colon adenocarcinoma was transplanted. Note that, in the term "αSQAP Cm:n", "α" represents an α anomer, "β" represents a β anomer, and "Cm:n" means that the number of carbon atoms and the number of double bonds in an acyl residue of SQAP are "m" and "n", respectively.

### SUMMARY OF THE INVENTION

The present invention in its one aspect provides a pharmaceutical composition as specified in clams 1 to 17.

### DESCRIPTION OF THE EMBODIMENTS

Patent Literatures above do not disclose a method in which a pharmaceutical composition is administered in combination with an anti-cancer agent for cancer treatment.

An embodiment of the present invention provides an anti-cancer agent whose effect is potentiated.

Hereinafter, embodiments will be described in detail with reference to the attached drawings. Note, the following embodiments are not intended to limit the scope of the claimed invention, and limitation is not made to an invention that requires a combination of all features described in the embodiments. Two or more of the multiple features described in the embodiments may be combined as appropriate. Furthermore, the same reference numerals are given to the same or similar configurations, and redundant description thereof is omitted.

A pharmaceutical composition for cancer treatment according to one embodiment of the present invention is administered to a patient with cancer in combination with an anti-cancer agent. The pharmaceutical composition contains a compound represented by General Formula (I) or a pharmaceutically acceptable salt thereof. In this specification, a compound represented by Formula (I) or a pharmaceutically acceptable salt thereof is referred to as a sulfoquinovosylacylpropanediol derivative. One embodiment of the present invention is a method of treating cancer, including administering a compound represented by General Formula (I) or a pharmaceutically acceptable salt thereof to a patient with cancer in combination with an anti-cancer agent.

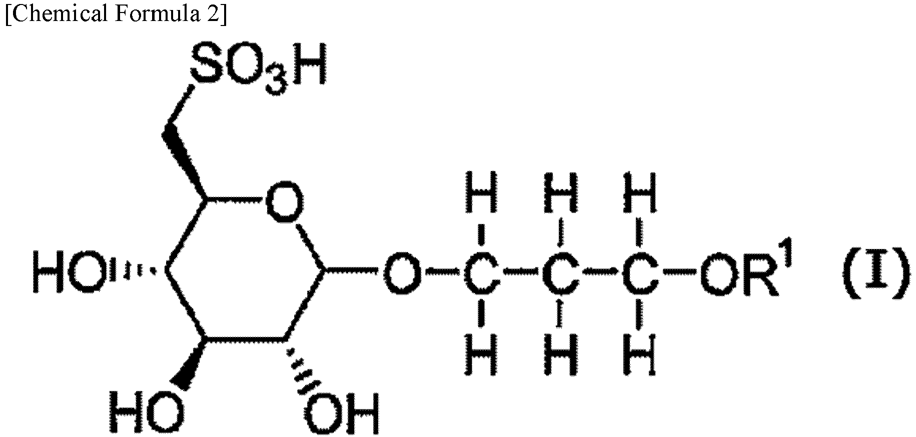

In Formula (I), R¹ is an acyl residue from a fatty acid. The acyl residue from a fatty acid corresponds to a residue obtained by removing OH from a carboxyl group of a fatty acid. There is no particular limitation on the type of fatty acid, and a linear or branched, saturated or unsaturated fatty acid may be employed. Also, there is no particular limitation on the number of carbon atoms in the fatty acid, and it is sufficient that the number of carbon atoms is 1 or more, but the number of carbon atoms is preferably 5 or more (medium-chain or longer fatty acids), more preferably 6 or more, even more preferably 10 or more, even more preferably 12 or more (long-chain fatty acids), and particularly preferably 16 or more. Meanwhile, the number of carbon atoms is preferably 26 or less, more preferably 22 or less, even more preferably 20 or less, and even more preferably 18 or less. The fatty acid is preferably a linear fatty acid, and more preferably a linear saturated fatty acid.

Examples of the fatty acid include caprylic acid, capric acid, lauric acid, myristic acid, palmitic acid, palmitoleic acid, stearic acid, oleic acid, vaccenic acid, linoleic acid, linolenic acid, eleostearic acid, arachidic acid, mead acid, arachidonic acid, behenic acid, lignoceric acid, nervonic acid, and cerotic acid.

There is no particular limitation on the steric arrangement of a propanediol moiety (-OCH₂CH₂CH₂OR¹) on a quinovose ring. That is to say, the sulfoquinovosylacylpropanediol derivative may be an α anomer (the sulfomethyl group and the propanediol moiety are arranged in a trans conformation), a β anomer (the sulfomethyl group and the propanediol moiety are arranged in a cis conformation), or a mixture of the α anomer and the β anomer. The sulfoquinovosylacylpropanediol derivative is preferably an α anomer. Note that the quinovose ring may have a boat conformation or chair conformation, or may be a mixture of a boat-conformation type and a chair-conformation type.

The pharmaceutically acceptable salt of the compound represented by Formula (I) refers to a salt that can be administered to a patient. That is to say, the compound represented by Formula (I) may form a salt with a positive ion, and, for example, the sulfo group of the compound represented by Formula (I) includes a positive ion instead of a hydrogen ion. In this case, the sulfo group of the compound represented by Formula (I) is represented as -SO₂A, where A represents a positive ion.

There is no particular limitation on the type of pharmaceutically acceptable salt. For example, the pharmaceutically acceptable salt may be a metal salt with a metal ion or an organic salt with an organic positive ion. Examples of the pharmaceutically acceptable salt of the compound represented by Formula (I) include salts with a monovalent positive ion such as a sodium salt and a potassium salt, and salts with a divalent positive ion such as a calcium salt and a magnesium salt.

It is needless to say that the present invention also encompasses prodrugs that form a compound represented by Formula (I) or a pharmaceutically acceptable salt thereof in a formulation, or when a formulation is diluted for administration, or in the body of a patient. Examples of such prodrugs include esters formed by the formation of a bond between a hydroxyl group linked to a quinovose ring and a carboxylic acid. It is needless to say that the present invention also encompasses hydrates of a compound represented by Formula (I) or a pharmaceutically acceptable salt thereof.

The sulfoquinovosylacylpropanediol derivative can be manufactured in accordance with, for example, the method disclosed in Patent Literature 3.

A pharmaceutical composition according to one embodiment of the present invention contains an effective amount of the sulfoquinovosylacylpropanediol derivative as an active component. A pharmaceutical composition according to one embodiment of the present invention may contain two or more types of sulfoquinovosylacylpropanediol derivatives. For example, the pharmaceutical composition may contain two or more types of sulfoquinovosylacylpropanediol derivatives that are salts different from each other, or two or more types of sulfoquinovosylacylpropanediol derivatives that are different from each other in the substituent R¹.

Furthermore, a pharmaceutical composition according to one embodiment of the present invention may contain components other than the sulfoquinovosylacylpropanediol derivative. Examples of such components include other pharmaceutical compositions, radiosensitizing agents, anti-tumor agents, and other substances having pharmacological activity. That is to say, a pharmaceutical composition according to one embodiment of the present invention can function as a radiosensitizing agent. In addition, other examples of such components include substances having no pharmacological activity such as additives, stabilizers, vehicles, and diluents.

A pharmaceutical composition according to one embodiment of the present invention is administered to a patient with cancer in combination with an anti-cancer agent. Examples of the anti-cancer agent used in combination with a pharmaceutical composition according to one embodiment of the present invention include: anti-cancer antibiotics such as mitomycin C, doxorubicin, bleomycin, and MMC; topoisomerase inhibitors such as camptothecin; microtubule polymerization inhibitors such as etoposide and taxol; alkylating agents such as MMS (methyl methanesulfonate); and platinum-containing drugs such as cisplatin.

Hereinafter, use of the pharmaceutical composition containing the sulfoquinovosylacylpropanediol derivative and a method of treating cancer in which this pharmaceutical composition is used will be described. The sulfoquinovosylacylpropanediol derivative can be used as a pharmaceutical composition for cancer treatment. A pharmaceutical composition according to one embodiment of the present invention refers to a drug that is administered to a patient in combination with an anti-cancer agent and improves the anti-cancer effect.

A pharmaceutical composition according to one embodiment of the present invention may also be administered to a patient with cancer in combination with radiation treatment in addition to an anti-cancer agent. In the radiation treatment, cancer is irradiated with radiation for the purpose of reducing the size of the cancer or eliminating the cancer. Here, as described above, a pharmaceutical composition according to one embodiment of the present invention can function as, for example, a radiosensitizing agent.

There is no particular limitation on the type of animal (patient) to be treated. Examples of the animal to be treated include mammals such as humans, dogs, cats, and horses. In one embodiment, the animal to be treated is a dog or cat from the viewpoint of obtaining higher efficacy. That is to say, a pharmaceutical composition according to one embodiment of the present invention is a pharmaceutical composition for cancer treatment that contains a compound represented by General Formula (I) or a pharmaceutically acceptable salt thereof and that is to be administered to a dog or cat. Radiation treatment in which a pharmaceutical composition according to one embodiment is used exhibits a therapeutic effect of reducing the size of or eliminating cancer developed in the body of a patient.

There is no particular limitation on the type of cancer to be treated. Examples of the type of cancer include neurogenic tumors such as brain tumor, carcinomas such as squamous cell carcinoma and adenocarcinoma (e.g., head and neck cancer, skin cancer, esophageal cancer, thyroid cancer, gastric cancer, lung cancer, gallbladder cancer, biliary tract cancer, pancreatic cancer, liver cancer, prostate cancer, uterine cancer, ovarian cancer, breast cancer, renal cancer, bladder cancer, and large bowel cancer), melanoma, bone/soft tissue tumors, lymphoma, leukemia, and myeloma.

In one embodiment, the type of cancer is skin cancer or blood cancer, particularly melanoma or lymphoma, from the viewpoint of obtaining higher efficacy. In another embodiment, the type of cancer is squamous cell carcinoma or adenocarcinoma from the viewpoint of obtaining higher efficacy. In yet another embodiment, the type of cancer is intraoral tumor or intranasal tumor from the viewpoint of obtaining higher efficacy.

In one embodiment, the pharmaceutical composition is used such that a compound represented by General Formula (I) is administered to a patient in an amount resulting in the blood concentration of 60 µM or less in the patient. That is to say, a pharmaceutical composition according to one embodiment is a pharmaceutical composition for cancer treatment that contains a compound represented by General Formula (I) or a pharmaceutically acceptable salt thereof and that is used such that the compound represented by General Formula (I) is administered to a patient in an amount resulting in the blood concentration of 60 µM or less in the patient. The compound represented by General Formula (I) is preferably administered to a patient at a dose of 50 µM or less, preferably 45 µM or less, preferably 42.5 µM or less, preferably 40 µM or less, preferably 37.5 µM or less, preferably 35 µM or less, preferably 32.5 µM or less, preferably 30 µM or less, and more preferably 25 µM or less.

In one embodiment, the pharmaceutical composition is used such that a compound represented by General Formula (I) is administered to a patient at a dose of 15 mg/kg or less. That is to say, a pharmaceutical composition according to one embodiment is a pharmaceutical composition for cancer treatment that contains a compound represented by General Formula (I) or a pharmaceutically acceptable salt thereof and that is used such that the compound represented by General Formula (I) is administered to a patient at a dose of 15 mg/kg or less. The compound represented by General Formula (I) is preferably administered to a patient at a dose of 10 mg/kg or less, preferably 8 mg/kg or less, preferably 5 mg/kg or less, preferably 3 mg/kg or less, more preferably 2 mg/kg or less, and even more preferably 1 mg/kg or less. Selecting such a dosage makes it possible to obtain sufficient efficacy while increasing a dose.

In one embodiment, the pharmaceutical composition is used such that a compound represented by General Formula (I) is administered to a patient in an amount resulting in the blood concentration of 1 µM or more in the patient. That is to say, a pharmaceutical composition according to one embodiment is a pharmaceutical composition for cancer treatment that contains a compound represented by General Formula (I) or a pharmaceutically acceptable salt thereof and that is used such that a compound represented by General Formula (I) is administered to a patient in an amount resulting in the blood concentration of 1 µM or more in the patient. The compound represented by General Formula (I) is preferably administered to a patient in an amount resulting in the blood concentration of 5 µM or more, preferably 10 µM or more, preferably 15 µM or more, preferably 20 µM or more, preferably 25 µM or more, preferably 27.5 µM or more, preferably 30 µM or more, preferably 32.5 µM or more, preferably 35 µM or more, preferably 37.5 µM or more, preferably 40 µM or more, and more preferably 42.5 µM or more, in a patient.

The pharmaceutical composition is used such that a compound represented by General Formula (I) is administered to a patient at a dose of 0.1 mg/kg or more, preferably 0.2 mg/kg or more, preferably 0.5 mg/kg, and preferably 1 mg/kg or more. The compound represented by General Formula (I) is preferably administered to a patient at a dose of more than 2 mg/kg, more preferably 3 mg/kg or more, and even more preferably 4 mg/kg or more. Selecting such a dosage makes it possible to obtain sufficient efficacy while reducing a dose.

A pharmaceutical composition according to one embodiment of the present invention may be administered, for example, simultaneously with an anti-cancer agent, or to a patient to which an anti-cancer agent has been administered, or to a patient to which an anti-cancer agent is to be administered, and there is no particular limitation on specific timing at which the pharmaceutical composition and the anti-cancer agent are administered. For example, the pharmaceutical composition may be administered every day while the anti-cancer agent may be administered periodically (e.g., every three weeks). Also, for example, the pharmaceutical composition and the anti-cancer agent may be administered on the same day.

In one embodiment, the administration of the pharmaceutical composition and the administration of the anti-cancer agent are performed as a set, and treatment that includes this set is repeated. There is no limitation on the frequencies of the administration of the pharmaceutical composition and the administration of the anti-cancer agent included in this set. For example, the set may include administering a dose of the pharmaceutical composition before and after the administration of the anti-cancer agent. Also, for example, the set may include administering a dose of the anti-cancer agent and three doses of the pharmaceutical composition at regular intervals over three weeks. This set is preferably repeated, for example, three or more times, preferably four or more times, and more preferably five or more times. Repeating a set of administration as described above makes it possible to obtain higher efficacy. There is no particular limitation on how many times this set is repeated, and this set is preferably repeated, for example, ten or less times, preferably eight or less times, and more preferably six or less times. With this embodiment, higher efficacy can be obtained while side effects of the anti-cancer agent are suppressed.

In one embodiment, the timing at which the pharmaceutical composition is administered need not correspond to the timing at which the anti-cancer agent is administered. For example, in the case of administering radiation treatment together, the pharmaceutical composition may be administered right before the radiation treatment. Here, the wording "the pharmaceutical composition is administered right before the radiation treatment" means that the pharmaceutical composition is administered up to 3 hours before the radiation treatment is started.

The frequency of the administration of the anti-cancer agent can be set as appropriate according to the type of anti-cancer agent, the administration method, or the like. For example, in the case of administering the anti-cancer agent every day, the pharmaceutical composition may be administered right before the administration of the anti-cancer agent. Here, the wording "the pharmaceutical composition is administered right before the administration of the anti-cancer agent" means that the pharmaceutical composition is administered up to 1 hour before the administration of the anti-cancer agent. The anti-cancer agent is preferably administered within 30 minutes, and more preferably 10 minutes, after the administration of the pharmaceutical composition.

The other administration conditions of the pharmaceutical composition and the other administration conditions of the anti-cancer agent can be selected as appropriate by healthcare workers and other professionals depending on the type, administration route, and administration timing of the pharmaceutical composition; the type, administration route, and administration timing of the anti-cancer agent; the type of disease to be treated and the severity of the disease; the age, weight, health state, and case history of a patient to be treated; and the like.

There is no particular limitation on the administration route of the pharmaceutical composition. Examples of the administration route include oral administration and parenteral administration. A suitable dosage form for oral administration is a solid dosage form, a semi-solid dosage form, a liquid dosage form, or a gaseous dosage form, and specific examples thereof include, but are not limited to, a tablet, a capsule, a powdered medicine, a granular medicine, a liquid medicine, a suspension, a syrup, an elixir, and an aerosol. Examples of a parenteral administration method include injection, dermal administration, rectal administration, and intraocular administration. Examples of an injection administration method include subcutaneous administration, intracutaneous administration, intravenous administration, and intramuscular administration.

In one embodiment, the pharmaceutical composition is intravenously administered from the viewpoint of obtaining higher efficacy. Moreover, it is preferable to slowly administer the pharmaceutical composition over 1 minute or longer from the viewpoint of suppressing local inflammation.

There is no particular limitation on a radiation treatment method that is performed in combination with the administration of a pharmaceutical composition according to one embodiment of the present invention in cancer treatment. For example, the same radiation type, irradiation amount, and irradiation frequency as those employed in conventional radiation treatment can be employed. In an example of the radiation treatment method, medical radiation such as X rays, γ rays, electron rays, β rays, corpuscular rays (e.g., π-mesons, neutrons, proton beams, or other baryons), brachytherapy, or nuclear medical treatment (RI oral administration therapy) is applied over a period of 1 day to 6 months at a dose of about 0.1 to 100 Gy (about 1 to 500 Gy in total).

As described above, administering an effective amount of the pharmaceutical composition to a patient in need thereof in combination with the anti-cancer agent makes it possible to treat cancer in the patient.

### [Examples]

An anti-cancer agent and a pharmaceutical composition were administered to test cells and then the cells were cultured. Then, after colonies were formed, the number of colonies was counted. Here, samples in which the concentration of the anti-cancer agent to be administered was varied were prepared, and in each sample, the survival fraction of the cells compared with the case where cells were cultured without administration of the pharmaceutical composition was measured. Regarding the type of anti-cancer agent to be administered, a predetermined anti-cancer agent was selected depending on the type of administration target cells. Note that cells that were cultured without administration of the pharmaceutical composition and the anti-cancer agent were used as a control. Hereinafter, these steps will be described.

A compound A (sulfoquinovosylacylpropanediol, SQAP) was used as the pharmaceutical composition. Here, SQAP is a dimer of an α anomer (molecular weight: about 1175) that includes CO(CH₂)₁₆CH₃ (stearoyl group: acyl residue from stearic acid) as R¹ in Formula (I).

Here, CHO cells, V79 cells, and canine-derived cancer cells were prepared as the test cells. The CHO cells are ovarian cells derived from Chinese hamsters, and the V79 cells are fibroblasts derived from the lungs of Chinese hamsters. Melanoma developing cells (CML6M) and soft tissue sarcoma developing cells (STSA-1) were used as the cancer cells.

The cancer cells were prepared by being cultured using an αMEM culture medium supplemented with 10% serum and an antibiotic. The cancer cells were prepared such that, when the concentration of the cells in a cell suspension obtained by subjecting the cultured cells to PBS washing and trypsin treatment, the concentration was within a predetermined concentration range. Here, the predetermined concentration ranges were 300 cells/well, 100 to 150 cells/well, and 500 to 1000 cells/well for the CHO/V79 cells, the melanoma cells, and the soft tissue sarcoma cells, respectively.

Next, the cancer cells were cultured in culture environments to which the anti-cancer agent was added at various concentrations, and were then allowed to form colonies. The culture periods for colony formation were a week, 10 days, and 14 days for CHO and V79, melanoma, and soft tissue sarcoma, respectively. After colonies were formed, washing using a physiological saline solution, fixation using 100% ethanol, and staining using 0.1% crystal violet were performed, and then the number of colonies per well was counted under a microscope. Note that, here, the colony was defined as containing 50 or more cells. Moreover, mitomycin C, doxorubicin, bleomycin,, camptothecin, etoposide, taxol, methyl methanesulfonate, and cisplatin were used as the anti-cancer agent.

A graph with the horizontal axis representing the concentration of the anti-cancer agent and the vertical axis representing the survival fraction was created by plotting the survival fraction measurement results relative to the SQAP administration amounts, and the effect of the addition of SQAP on the survival fraction was examined. Hereinafter, the results from each anti-cancer agent will be shown in a table and described.

### (Example 1)

CHO cells were used as the cells, and mitomycin C was used as the anti-cancer agent. The amount of the mitomycin C was set to 0 nM, 100 nM, 200 nM, 300 nM, 400 nM, and 500 nM. Table 1 below shows the survival fraction in the case where SQAP was administered at a concentration of 30 µM and in the case where SQAP was not administered. In Table 1, survival fractions in the case where SQAP was not administered are shown with a solid line, and survival fractions in the case where SQAP was administered at a concentration of 30µM are shown with a broken line.

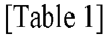

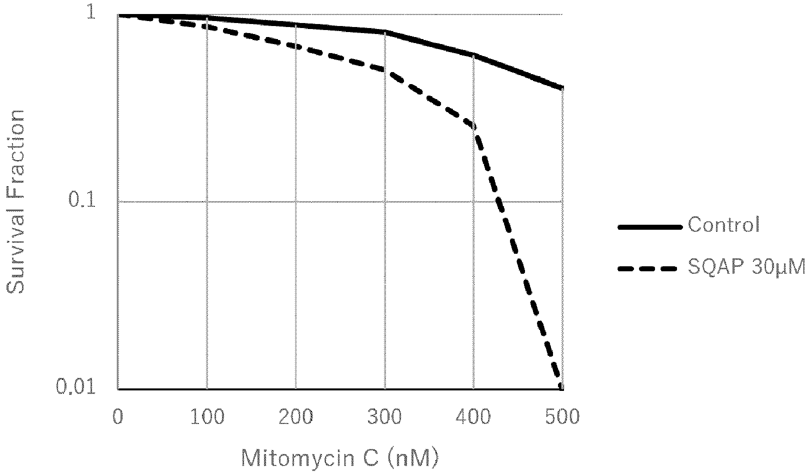

When the mitomycin C was administered to the CHO cells at a concentration of 500 nM and the SQAP was not administered thereto, the survival fraction was about 40%. Meanwhile, when the SQAP was administered to the CHO cells in the same conditions at a concentration of 30 µM, the survival fraction was about 1%, and it was confirmed that the survival fraction significantly decreased compared with the case where SQAP was not administered.

### (Example 2)

Canine-derived melanoma cells and soft tissue sarcoma cells were used as the cells, and doxorubicin was used as the anti-cancer agent. The amount of the doxorubicin administered to the melanoma cell was set to 0 nM, 5 nM, 10 nM, 15 nM, 20 nM, and 25 nM. Also, the concentration of the doxorubicin administered to the soft tissue sarcoma cells was set to 0 nM, 5 nM, 10 nM, and 15 nM. Table 2 below shows the survival fractions in the case where SQAP was administered at concentrations of 10 µM, 20 µM, and 30 µM and in the case where SQAP was not administered. In Table 2, survival fractions in the case where SQAP was not administered are shown with a solid line, survival fractions in the case where SQAP was administered at a concentration of 10µM are shown with a broken line, survival fractions in the case where SQAP was administered at a concentration of 20µM are shown with a 1-dot chain line, and survival fractions in the case where SQAP was administered at a concentration of 30µM are shown with a 2-dot chain line.

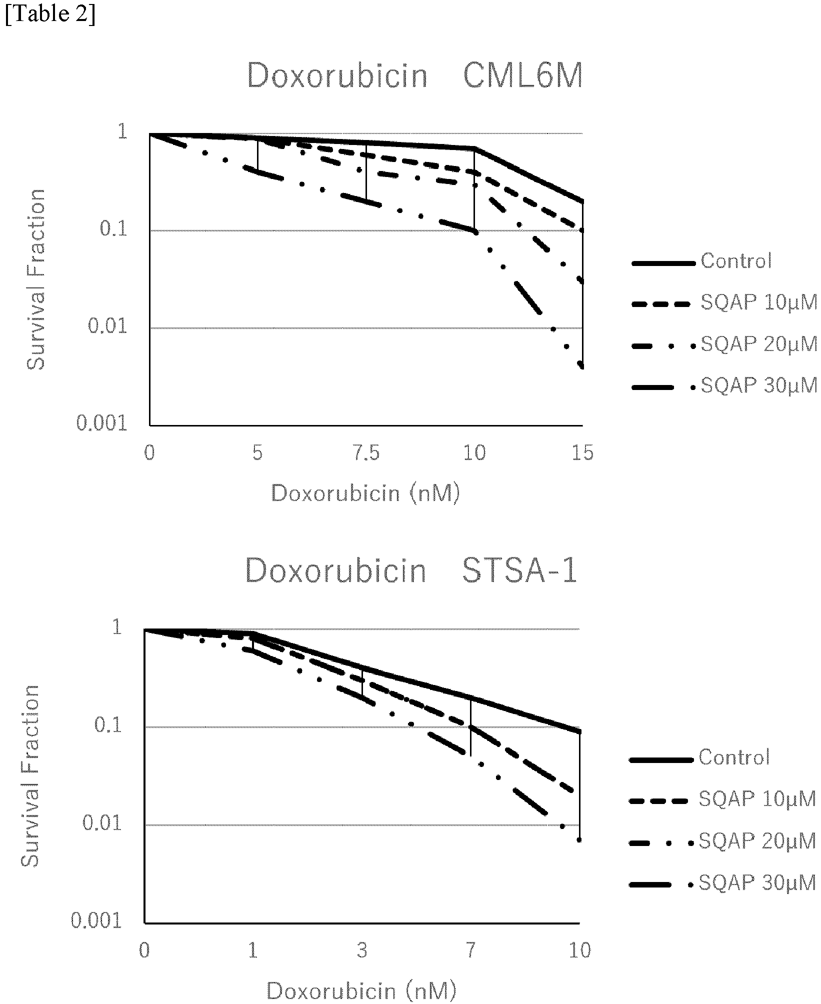

When the doxorubicin was administered to the melanoma cells at a concentration of 15 nM and the SQAP was not administered thereto, the survival fraction was about 20%. Meanwhile, when the SQAP was administered to the melanoma cells in the same conditions at concentrations of 10 µM, 20 µM, and 30 µM, the survival fractions were about 10%, about 3%, and about 0.4%, respectively. Also, when the doxorubicin was administered to the soft tissue sarcoma cells at a concentration of 10 nM and the SQAP was not administered thereto, the survival fraction was about 6%. Meanwhile, when the SQAP was administered to the soft tissue sarcoma cells in the same conditions at concentrations of 10 µM, 20 µM, and 30 µM, the survival fractions were about 2%, about 2%, and about 0.7%, respectively. As described above, it was confirmed that, when the doxorubicin was administered and the SQAP was also administered, the survival fraction significantly decreased depending on the administration amount compared with the case where the SQAP was not administered.

### (Example 3)

V79 cells were used as the cells, and bleomycin was used as the anti-cancer agent. The bleomycin was administered at concentrations of 0 µg/mL, 5 µg/mL, 10 µg/mL, and 15 µg/mL. Table 3 below shows the survival fractions in the case where SQAP was administered at a concentration of 20 µM and in the case where SQAP was not administered.

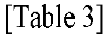

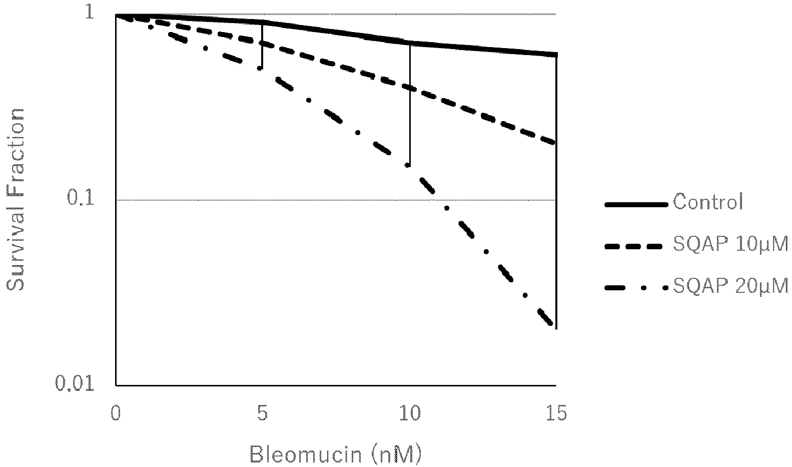

When the bleomycin was administered to the cells at a concentration of 15 nM and the SQAP was not administered thereto, the survival fraction was about 40%. Meanwhile, when the SQAP was administered to the cells in the same conditions at concentrations of 10 µM and 20 µM, the survival fractions were about 20% and about 2%, respectively. As described above, it was confirmed that, when the bleomycin was administered and the SQAP was also administered, the survival fraction significantly decreased depending on the administration amount compared with the case where the SQAP was not administered.

### (Example 4)

V79 cells were used as the cells, and cisplatin was used as the anti-cancer agent. The cisplatin was administered at concentrations of 0 µg/mL, 0.1 µg/mL, 0.2 µg/mL, 0.3 µg/mL, 0.4 µg/mL, and 0.5 µg/mL. Table 4 below shows the survival fractions in the case where SQAP was administered at concentrations of 10 µM and 20 µM and in the case where SQAP was not administered.

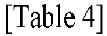

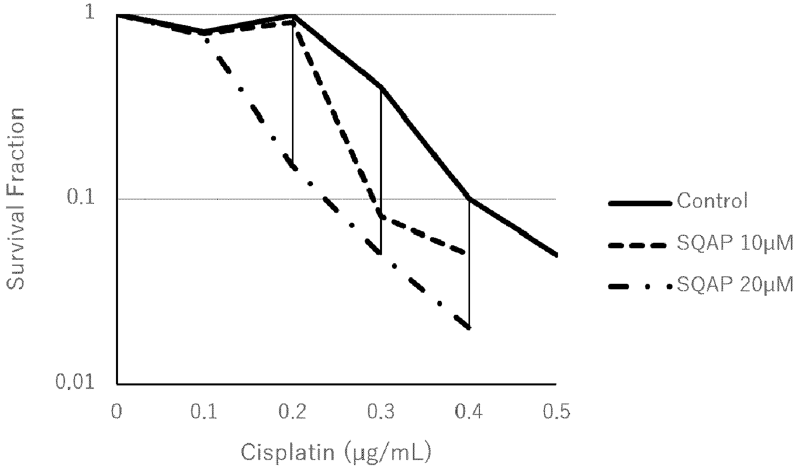

When the cisplatin was administered to the cells at a concentration of 0.3 µg/mL and the SQAP was not administered thereto, the survival fraction was about 40%. Meanwhile, when the SQAP was administered to the cells in the same conditions at concentrations of 10 µM and 20 µM, the survival fractions were about 8% and about 5%, respectively. As described above, it was confirmed that, when the cisplatin was administered and the SQAP was also administered, the survival fraction significantly decreased depending on the administration amount compared with the case where the SQAP was not administered.

### (Example 5)

V79 cells were used as the cells, and camptothecin was used as the anti-cancer agent. The amount of the camptothecin was set to 0 nM, 5 nM, 10 nM, 15 nM, and 20 nM. Table 5 below shows the survival fractions in the case where SQAP was administered at concentrations of 10 µM and 20 µM and in the case where SQAP was not administered.

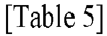

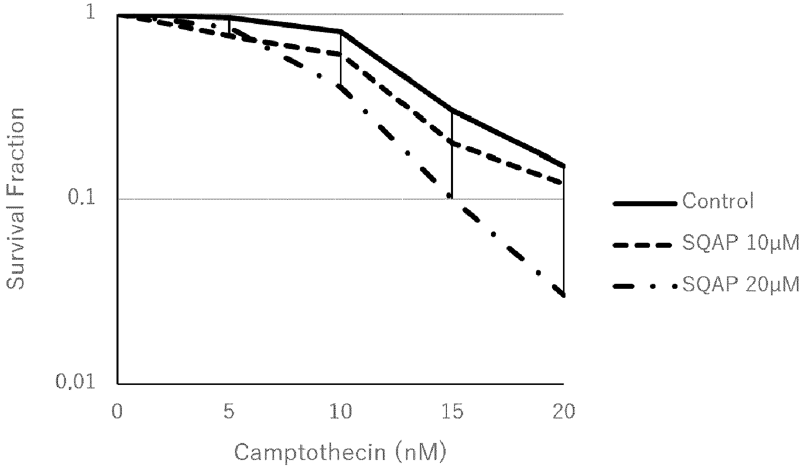

When the camptothecin was administered to the cells at a concentration of 20 nM and the SQAP was not administered thereto, the survival fraction was about 15%. Meanwhile, when the SQAP was administered to the cells in the same conditions at concentrations of 10 µM and 20 µM, the survival fractions were about 12% and about 3%, respectively. As described above, it was confirmed that, when the camptothecin was administered and the SQAP was also administered, the survival fraction significantly decreased depending on the administration amount compared with the case where the SQAP was not administered.

### (Example 6)

V79 cells were used as the cells, and mitomycin C was used as the anti-cancer agent. The amount of MMC was set to 0 nM, 100 nM, 200 nM, 300 nM, 400 nM, and 500 nM. Table 6 below shows the survival fractions in the case where SQAP was administered at concentrations of 10 µM and 20 µM and in the case where SQAP was not administered.

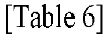

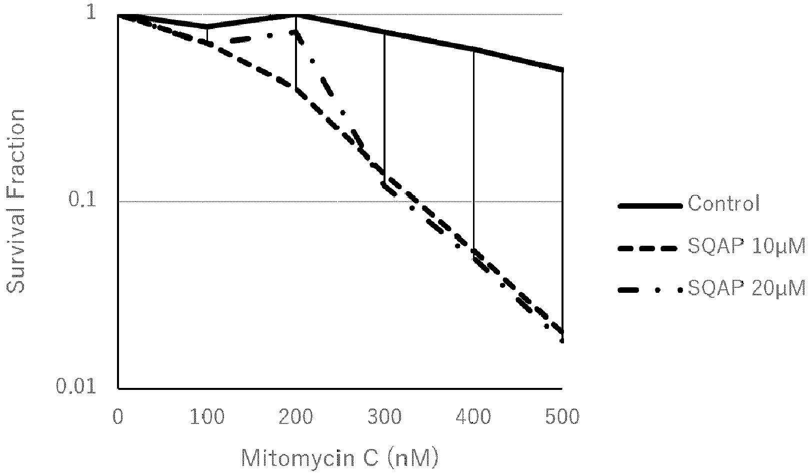

When the mitomycin C was administered to the cells at a concentration of 500 nM and the SQAP was not administered thereto, the survival fraction was about 50%. Meanwhile, when the SQAP was administered to the cells in the same conditions at concentrations of 10 µM and 20 µM, the survival fractions were about 2% and about 1.8%, respectively. As described above, it was confirmed that, when the MMC was administered and the SQAP was also administered, the survival fraction significantly decreased depending on the administration amount compared with the case where the SQAP was not administered.

### (Example 7)

V79 cells were used as the cells, and etoposide was used as the anti-cancer agent. The amount of the etoposide was set to 0 µM, 5 µM, 10 µM, 15 µM, 20 µM, 25 µM, and 30 µM. Table 6 below shows the survival fractions in the case where SQAP was administered at concentrations of 10 µM and 20 µM and in the case where SQAP was not administered.

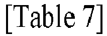

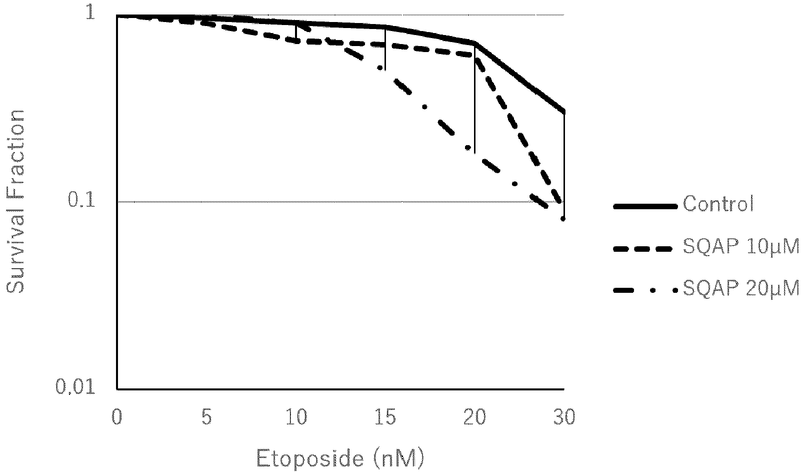

When the etoposide was administered to the cells at a concentration of 30 nM and the SQAP was not administered thereto, the survival fraction was about 30%. Meanwhile, when the SQAP was administered to the cells in the same conditions at concentrations of 10 µM and 20 µM, the survival fractions were about 9% and about 8%, respectively. As described above, it was confirmed that, when the etoposide was administered and the SQAP was also administered, the survival fraction significantly decreased depending on the administration amount compared with the case where the SQAP was not administered.

### (Example 8)

V79 cells were used as the cells, and taxol was used as the anti-cancer agent. The amount of the taxol was set to 0nM, 10nM, 20nM, and 30nM. Table 8 below shows the survival fractions in the case where SQAP was administered at concentrations of 10 µM and 20 µM and in the case where SQAP was not administered.

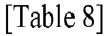

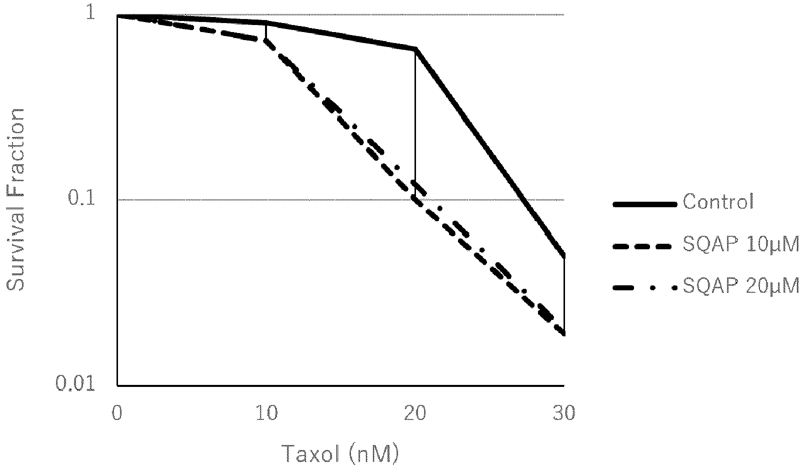

When the taxol was administered to the cells at a concentration of 30 nM and the SQAP was not administered thereto, the survival fraction was about 5%. Meanwhile, when the SQAP was administered to the cells in the same conditions at concentrations of 10 µM and 20 µM, the survival fractions were about 2% in both cases. As described above, it was confirmed that, when the taxol was administered and the SQAP was also administered, the survival fraction decreased depending on the administration amount compared with the case where the SQAP was not administered.

### (Example 9)

V79 cells were used as the cells, and MMS was used as the anti-cancer agent. The amount of the MMS was set to 0 µM, 10 µM, 20 µM, 30 µM, and 40 µM. Table 9 below shows the survival fractions in the case where SQAP was administered at concentrations of 10 µM and 20 µM and the case where SQAP was not administered.

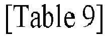

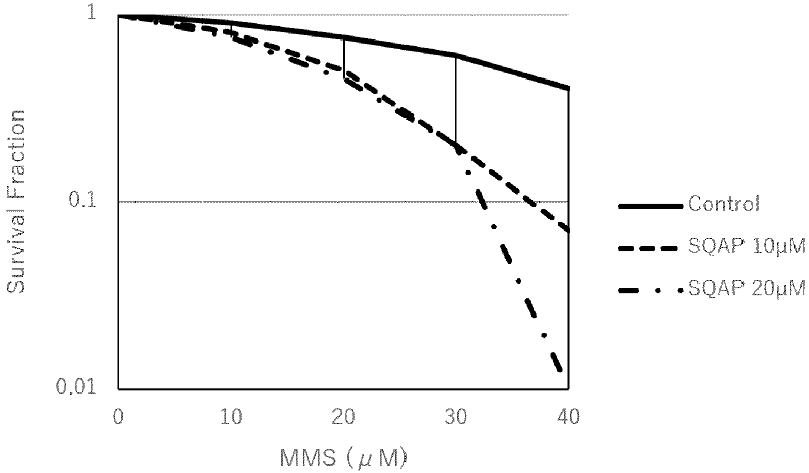

When the MMS was administered to the cells at a concentration of 40µM and the SQAP was not administered thereto, the survival fraction was about 40%. Meanwhile, when the SQAP was administered to the cells in the same conditions at concentrations of 10 µM and 20 µM, the survival fractions were about 7% and less than 1%, respectively. As described above, it was confirmed that, when the MMS was administered and the SQAP was also administered, the survival fraction significantly decreased depending on the administration amount compared with the case where the SQAP was not administered.

(Example 10) In the above examples, the anti-cancer agent and the pharmaceutical composition were administered to the test cells and then the cells were cultured. In this example, test cells were cultured after administering pharmaceutical composition (SQAP) alone without administering the anti-cancer agent to the test cells, and then the number of colonies was counted after colonies were formed. The culture and the count of the number of colonies were carried out in the same manner as in Examples 1 to 9, except not to administering the anti-cancer agent.

CHO cells, melanoma developing cells (CML6M), soft tissue sarcoma developing cells (STSA-1), and V79 cells were prepared as the test cells, which SQAP was administered to. SQAP was administered at 30µM to CHO cells, at 10µM, 20µM and 30µM to melanoma developing cells and soft tissue sarcoma developing cells, and at 10µM and 20µM to V79 cells respectively.

The survival fraction was about 1% in the case where the mitomycin C at a concentration of 500µM and SQAP at a concentration of 30µM was administered to the cells, while the survival fraction was about 75% in the case where SQAP at a concentration of 30µM was administered to the cells alone. The survival fractions were about 10%, about 3%, and about 0.4%, respectively, in the cases where doxorubicin (at a concentration of 10µM) and the SQAP (at a concentration of 10µM, 20µM and 30µM respectively) were administered to the melanoma developing cells. Meanwhile, the survival fractions were about 90%, about 85%, and about 60%, respectively, in the cases where SQAP (at a concentration of 10µM, 20µM and 30µM respectively) was administered to the melanoma developing cells alone. The survival fractions were about 2%, about 2%, and about 0.7%, respectively, in the cases where doxorubicin (at a concentration of 10µM) and the SQAP (at a concentration of 10µM, 20µM and 30µM respectively) were administered to the soft tissue sarcoma developing cells. Meanwhile, the survival fractions were about 90%, about 75%, and about 45%, respectively, in the cases where SQAP (at a concentration of 10µM, 20µM and 30µM respectively) was administered to the soft tissue sarcoma developing cells alone. The survival fractions were about 8%, and about 5%, respectively, in the case where cisplatin (at a concentration of 0.3pg/mL) and the SQAP (at a concentration of 10µM, and 20µM respectively) were administered to the V79 cells. Meanwhile, the survival fractions were 10% in either case, in the cases where SQAP (at a concentration of 10µM, and 20µM respectively) was administered to the V79 cells alone. Furthermore, in any case where the anti-cancer agent and SQAP were administered to the V79 in combination, which was shown in the above examples, the survival fraction of cells after culture decreased in the case where SQAP and the anti-cancer agent were administered in combination compared with the case where SQAP was administered alone. As described above, it was confirmed that the survival fraction significantly decreased in the case where the anti-cancer agent and SQAP were administered in combination compared with the case where SQAP was administered alone.

It was confirmed from the examples above that administering the pharmaceutical composition to cells in combination with an anti-cancer agent exerted a prominent effect of significantly reducing the survival fraction of cells damaged by the anti-cancer agent. Also, a significant effect that administering the pharmaceutical composition in combination with an anti-cancer agent lowered the survival fraction compared with the case where the pharmaceutical composition was not used in combination with an anti-cancer agent and was administered alone was confirmed. As described above, a pharmaceutical composition according to one embodiment of the present invention can potentiate the anti-tumor activity of an anti-cancer agent. Using a pharmaceutical composition in combination with an anti-cancer agent achieves a synergistic anti-tumor activity.

It is possible to potentiate the effect of an anti-cancer agent.

The invention is not limited to the foregoing embodiments, and various variations/changes are possible within the spirit of the invention.

## Claims

1. A pharmaceutical composition for cancer treatment to be administered to a patient with cancer in combination with an anti-cancer agent, the pharmaceutical composition comprising
a compound represented by General Formula (I) or a pharmaceutically acceptable salt thereof: 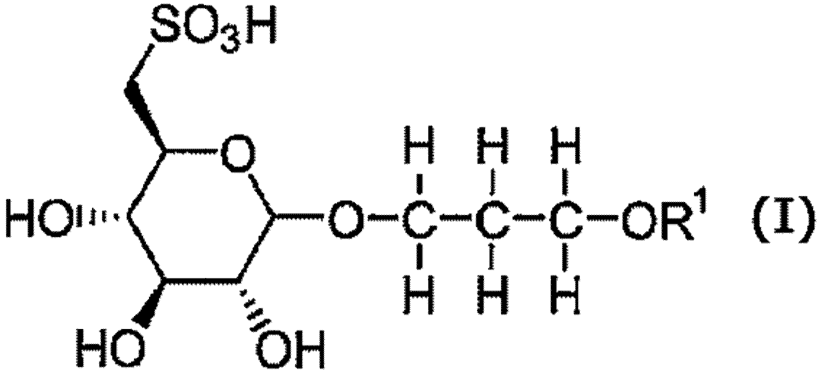
where R¹ is an acyl residue from a fatty acid.

2. The pharmaceutical composition according to claim 1,
wherein the patient is a mammal including a human, a dog, or a cat.

3. The pharmaceutical composition according to claim 1 or 2,
wherein the cancer is solid cancer.

4. The pharmaceutical composition according to claim 1 or 2,
wherein the cancer is melanoma, bone tumor, lymphoma, adenocarcinoma, or squamous cell carcinoma.

5. The pharmaceutical composition according to any one of claims 1 to 4, wherein the acyl residue has 1 to 26 carbon atoms.

6. The pharmaceutical composition according to any one of claims 1 to 5,
wherein R¹ is a stearoyl group.

7. The pharmaceutical composition according to any one of claims 1 to 6,
wherein the anti-cancer agent is a drug that exerts an anti-cancer effect by damaging DNA inside a cell, an alkylating agent, a platinum compound, or an anti-tumor antibiotic.

8. The pharmaceutical composition according to any one of claims 1 to 7,
wherein the anti-cancer agent is mitomycin C.

9. The pharmaceutical composition according to any one of claims 1 to 8,
which potentiates an anti-tumor activity of the anti-cancer agent.

10. The pharmaceutical composition according to any one of claims 1 to 9,
which is administered at a dosage for administering the compound represented by General Formula (I) or the pharmaceutically acceptable salt thereof in an amount resulting in a blood concentration of 60 µM or less in the patient.

11. The pharmaceutical composition according to any one of claims 1 to 9,
which is administered at a dosage for administering the compound represented by General Formula (I) or the pharmaceutically acceptable salt thereof in an amount of 15 mg/kg or less.

12. The pharmaceutical composition according to any one of claims 1 to 11,
which is administered at a dosage for administering the compound represented by General Formula (I) or the pharmaceutically acceptable salt thereof in an amount resulting in a blood concentration of 1 µM or more in the patient.

13. The pharmaceutical composition according to any one of claims 1 to 11,
which is administered at a dosage for administering the compound represented by General Formula (I) or the pharmaceutically acceptable salt thereof in an amount of 0.1 mg/kg or more.

14. The pharmaceutical composition according to any one of claims 1 to 13,
which is administered to the patient in combination with radiation treatment as well.

15. The pharmaceutical composition according to any one of claims 1 to 14,
wherein, in the anti-cancer agent treatment, administration of the composition represented by General Formula (I) or the pharmaceutically acceptable salt thereof and irradiation of the patient immediately following the administration are performed as a set, and this set is repeated, thereby irradiating cancer at a total dose of 2 Gy or more.

16. The pharmaceutical composition according to claim 15,
wherein the set is repeated at a frequency of once or less a day and once or more a month.

17. The pharmaceutical composition according to any one of claims 1 to 16, wherein the pharmaceutically acceptable salt includes a calcium salt or a sodium salt.
